# EUROPEAN PATENT APPLICATION

(11) **EP 4 311 566 A1**
(43) Date of publication of application: **31.01.2024**
(21) Application number: 22187663.4
(22) Date of filing: 28.07.2022
(51) Int. Cl.: A61M 15/00

(54) **AN INHALER COMPRISING A RETENTION ELEMENT**

(71) Applicant: Vectura Inc., Stamford, CT 06901-3721 (US)
(72) Inventor: SWANBURY, Philip John, Cambridge (GB); YADIDI, Kambiz, Beverly Hills (US); AYDIN, Mo, Stamford (US); GIBBS, Thomas Edward, Stamford (US)
(74) Representative: Clarke, Christopher John

(57) **Abstract**

There is provided an inhaler (200) comprising a housing defining an inlet (111), an outlet (113), and an airflow path extending from the inlet (111) to the outlet (113). The inhaler (200) comprises a capsule receptacle (221) for receiving a capsule (230) comprising a composition for inhalation. The inhaler (200) comprises a rupturing element (140) for rupturing the capsule (230) received in the capsule receptacle (221). The inhaler (200) comprises an aerosolization chamber (222) for allowing the composition to be entrained in airflow within the airflow path. The inhaler (200) comprises a retention element (250) movable between a first position in which the capsule (230) is prevented from moving from the capsule receptacle (221) to the aerosolization chamber (222), and a second position in which the capsule (230) is movable from the capsule receptacle (221) to the aerosolization chamber (222). There is also provided a composition that is administered to a patient using the inhaler (200).

## Description

The present disclosure relates to an inhaler. In particular, the present disclosure relates to an inhaler for use with a capsule comprising a composition. The present disclosure also relates to a method of administering a composition using the inhaler. The present disclosure further relates to a composition that is administered to a patient using the inhaler.

Inhalers are commonly used to deliver a pharmaceutically active ingredient to the lungs of a patient in order to treat various medical conditions which may include respiratory illnesses, such as asthma. Some medical conditions that can be treated by administering a pharmaceutically active ingredient via an inhaler may have an acute onset. These medical conditions may include a myocardial infarction, cerebrovascular accident, and asthma exacerbation. Thus, it is important that the inhaler is simple to use and can quickly and effectively deliver the pharmaceutically active ingredient to the patient. This may allow the best possible outcome for a patient experiencing an acute onset of a medical condition. However, even where a medical condition does not have an acute onset, it is important that the inhaler is simple to use and can quickly and effectively deliver the pharmaceutically active ingredient to the patient. For example, this may make it more likely that a patient persists with their treatment.

Some pharmaceutically active ingredients are better suited to being in the form of a liquid. Whereas, some other pharmaceutically active ingredients are better suited to being in the form of a solid, such as a dry powder.

In some known inhalers, the pharmaceutically active ingredient is in the form of a liquid contained within a pressurised container. The patient may actuate the inhaler to release the pharmaceutically active ingredient from the pressurised container. The pharmaceutically active ingredient is released in the form of an aerosol that can be inhaled and delivered to the patient's lungs.

In some other known inhalers, the pharmaceutically active ingredient is administered in the form of a dry powder that may be disposed within a capsule. Typically, the capsule is stored separately from the inhaler in blister packaging. Consequently, the patient must locate a capsule, extract the capsule from its packaging, open the inhaler to insert the capsule and then close the inhaler with the capsule in place. The patient may then actuate a piercing element to pierce the capsule and draw on the inhaler which causes the capsule to move within the inhaler and release the dry powder to form an aerosol that can be inhaled and delivered to the patient's lungs. In emergency situations, such as the onset of a thromboembolic event when time is crucial, these many steps could mean the difference between an effective or ineffective intervention. Typically, a patient must ensure that the inhaler is held upright after inserting the capsule. Otherwise, the capsule may move out of reach of the piercing element before the capsule can be pierced. It can be challenging for a patient to both insert the capsule and keep the inhaler upright or steady whilst experiencing a medical condition, particularly where the medical condition is debilitating and has an acute onset. Having to insert the capsule and keep the inhaler upright or steady can also increase the overall time it takes to deliver the pharmaceutically active ingredient to the patient.

It would be desirable to provide an inhaler for use with a capsule that may be easier for a patient to use compared to some other prior art inhalers. It would also be desirable to provide an inhaler for use with a capsule that may reduce the overall time to deliver the pharmaceutically active ingredient to the patient.

According to the present disclosure there is provided an inhaler. The inhaler comprises a housing defining an inlet, an outlet, and an airflow path extending from the inlet to the outlet. The inhaler comprises a capsule receptacle for receiving a capsule comprising a composition for inhalation. The inhaler comprises a rupturing element for rupturing the capsule received in the capsule receptacle. The inhaler comprises an aerosolization chamber for allowing the composition to be entrained in airflow within the airflow path. The inhaler comprises a retention element movable between a first position in which the capsule is prevented from moving from the capsule receptacle to the aerosolization chamber, and a second position in which the capsule is movable from the capsule receptacle to the aerosolization chamber.

The retention element prevents the capsule from moving from the capsule receptacle to the aerosolization chamber when the retention element is in the first position. This may keep the capsule within proximity of the rupturing element until a user desires to use the inhaler. This means that a capsule may be inserted into the inhaler at a point in time significantly prior to when a user desires to use the inhaler. The inhaler can then be carried in, for example, a pocket without fear of the capsule moving out of proximity of the rupturing element such that it cannot be ruptured. Advantageously, this may allow a user to be able to quickly treat their medical condition because the capsule can be preloaded. For example, the capsule may be preloaded in the morning but not be used until the evening.

The retention element may be moved to a position in which the capsule is movable from the capsule receptacle to the aerosolization chamber. Advantageously, this allows the patient to quickly activate the inhaler for use. Typically, the time and effort required to move the retention element from the first position to the second position is less than the time and effort required to locate a capsule and insert it into the inhaler. This is particularly beneficial during a time when the patient may be debilitated by a medical condition.

The inhaler comprises a capsule receptacle. Advantageously, this may ensure that the capsule is appropriately positioned with respect to the rupturing elements. For example, the capsule receptacle may position the capsule in an orientation that is optimum for rupturing the capsule. This optimum orientation may limit the force a user is required to apply to the rupturing element in order to rupture the capsule. Alternatively, or additionally, the optimum orientation may result in optimum rupturing of the capsule for releasing the composition.

As used herein, the term "rupturing" refers to providing at least one opening in the capsule for allowing the composition within the capsule to exit the capsule. Rupturing may include, but is not limited to: piercing, perforating, unscrewing, ripping and separating the capsule.

As used herein, the term "proximal end" refers to an end of the inhaler or a component of the inhaler that is nearest to the outlet. The term "distal end" refers to an end of the inhaler or component of the inhaler that is opposite the proximal end of the inhaler or component of the inhaler.

As used herein, the term "longitudinal" refers to a direction or axis extending between the proximal end and the distal end of the inhaler or component of the inhaler.

As used herein, the term "airflow" refers to any suitable gaseous flow for inhalation by a user and entraining the composition. For example, the gaseous flow may be a flow of air, a flow of oxygen or a flow of nitrous oxide.

As used herein, the term "pharmaceutically active ingredient" refers to an ingredient that alters one or more chemical or physiological functions of a cell, tissue, organ, or organism. A pharmaceutically active ingredient may be, for example, a systemic or local drug, a peptide or DNA based drug, an anti-inflammatory agent, a bronchodilating agent, an antiviral agent, an antibiotic agent, an immunostimulatory agent, an immunosupressive agent, an anesthetic agent, an anticancer agent, a vitamin, a hormone, an antiepileptic agent, an antifungal agent, an antioxidant, an antidiabetic agent, a muscle relaxant, and anti-HIV agent, a stimulant, a cough suppressant, a pain controller, a smoking cessation agent or an alcohol abuse agent.

As used herein, references to "a user" may also refer to "a patient".

The inhaler may be a dry powder inhaler.

The airflow path may extend through the aerosolization chamber. The airflow path may be configured to cause airflow within the aerosolization chamber to form a vortex within the aerosolization chamber. The vortexed airflow may cause a capsule to move from the capsule receptacle to the aerosolization chamber. The vortexed airflow may cause a capsule within the aerosolization chamber to rotate and release the composition into the airflow within the airflow path.

The airflow path may comprise an inlet channel extending from the inlet to the aerosolization chamber. The inlet channel may extend in a direction transverse to the longitudinal axis of the inhaler. The inlet channel may be configured to cause airflow within the aerosolization chamber to form a vortex within the aerosolization chamber. The inlet channel may be configured to direct airflow within the airflow channel towards a wall of the aerosolization chamber. The inlet channel may be configured to cause airflow within the airflow channel to enter the aerosolization chamber in direction substantially tangential to a wall of the aerosolization chamber.

The airflow path may comprise an outlet channel extending between the aerosolization chamber and the outlet.

The inlet may comprise a first inlet and a second inlet. The inlet channel may be a first inlet channel extending from the first inlet to the aerosolization chamber. The airflow path may comprise a second inlet channel extending from the second inlet to the aerosolization chamber. The second inlet channel may extend in a transverse direction to the aerosolization chamber. The second inlet channel may be configured to cause airflow within the aerosolization chamber to form a vortex within the aerosolization chamber. The second inlet channel may be configured to direct airflow within the airflow channel towards a wall of the aerosolization chamber. The second inlet channel may be configured to cause airflow to enter the aerosolization chamber in direction substantially tangential to a wall of the aerosolization chamber. The first inlet channel and the second inlet channel may cooperate to cause airflow within the aerosolization chamber to form a vortex within the aerosolization chamber.

The retention element may be separated from the housing when the retention element is in the second position. That is, the housing and the retention element are not in contact with one another when the retention element is in the second position. The retention element may be configured to separate from the housing when the retention element is moved from the first position to the second position. Advantageously, this may prevent the retention element from disrupting the airflow within the airflow path and thus limiting the amount of composition that is delivered to the user.

The inhaler may comprise an actuating means configured to move the retention element from the first position to the second position. Advantageously, the provision of an actuating means may allow the retention element to be quickly moved from the first position to the second position. The actuating means may be a mechanical actuating means. The actuating means may comprise a user-operable button operable to activate the actuating means. The user-operable button may be positioned at an external surface of the housing. Advantageously, such a user-operable button may make it easier for a user to move the retention element from the first position to the second position. The user-operable button may be a press button. The press button may be configured to be depressed to activate the actuating means. The user-operable button may be a slide button. The actuating means may be activated by airflow within the airflow path.

The retention element may be configured to move from the first position to the second position when a user inhales on the inhaler. Advantageously, this reduces the number of the steps a user is required to take in order to use the inhaler to inhale the composition.

The retention element may be configured such that airflow within the airflow path causes the retention element to move from the first position to the second position. For example, the retention element may be configured such that the pressure of airflow within the airflow path against the retention element causes the retention element to move from the first position to the second position. The retention element may be biased to the second position by a biasing element, such as a spring. A frangible element may retain the retention element in the first position. When a user inhales, the airflow within the airflow path may rupture the frangible element, thereby causing the biasing element to move the retention element from the first position to the second position.

The retention element may be configured to move from the first position to the second position when the rate of airflow within the airflow path is above a rate of airflow threshold. Advantageously, this may prevent the retention element from being prematurely moved from the first position to the second position in response to airflow through the airflow path that was not initiated by a user. The rate of airflow threshold may be between 10 L/min and 150 L/min, between 10 L/min and 130 L/min, between 10 L/min and 110 L/min, between 10 L/min and 90 L/min, between 10 L/min and 70 L/min, between 10 L/min and 50 L/min, between 10 L/min and 30 L/min or between 10 L/min and 20 L/min. Preferably, the rate of airflow threshold is between 10 L/min and 50 L/min. The rate of airflow threshold may be 10 L/min, 15 L/min, 20 L/min, 25 L/min, 30 L/min, 35 L/min, 40 L/min, 45 L/min or 50 L/min. Preferably, the rate of airflow threshold is 30 L/min (Litres per minute). For example, the frangible element may be configured to rupture when the rate of airflow is equal to or greater than the rate of airflow threshold.

The inhaler may be configured to prevent movement of the retention element from the first position to the second position until the capsule has been ruptured. Advantageously, this may ensure the retention element can only be moved from the first position to the second position when the user intends to use the inhaler to inhale the composition. For example, the retention element may only be able to move from the first position to the second position once the retention element has been moved to a rupturing position.

The retention element may comprise the rupturing element. Movement of the retention element from the first position to second position may be configured to cause the rupturing element to rupture the capsule. Advantageously, this reduces the number of the steps a user is required to take in order to use the inhaler to inhale the composition. For example, the rupturing element may be fixed to the retention element such that movement of the retention element from the first position to the second position causes movement of the rupturing element that ruptures the capsule.

Movement of the retention element from the first position to the second position may be configured to cause the capsule to move from the capsule receptacle to the aerosolization chamber. Advantageously, this may allow the composition to be delivered more quickly to a user. Movement of the retention element from the first position to the second position may be configured to cause the capsule to move from the capsule receptacle to the aerosolization chamber, and subsequently prevent the capsule from moving from the aerosolization chamber to the capsule receptacle.

The retention element may be configured to contact the capsule within the capsule receptacle when the retention element is in the first position. The retention element may be configured to substantially prevent movement of the capsule with respect to the capsule receptacle when the retention element is in the first position. The retention element may be configured to substantially prevent movement of the capsule with respect to the capsule receptacle when the retention element is in the first position by urging the capsule against a wall of the capsule receptacle. Advantageously, preventing movement of the capsule within the capsule receptacle may prevent the capsule from being damaged or prematurely ruptured. Furthermore, it may help to keep the capsule appropriately orientated with respect to the rupturing element.

The capsule receptacle may comprise a capsule receptacle opening for allowing a capsule to move between the capsule receptacle and the aerosolization chamber. The capsule receptacle opening may be disposed at an interface between the capsule receptacle and the aerosolization chamber. The retention element may be configured to at least partially cover the capsule receptacle opening when the retention element is the first position. The retention element may be configured to completely cover the capsule receptacle opening when the retention element is in the first position. The retention element may be configured to substantially prevent airflow through the airflow path from entering into the capsule receptacle when the retention element is in the first position. Advantageously, this may prevent composition in a ruptured capsule from prematurely being released before the retention element has been moved to the second position.

The retention element may comprise a first portion and a second portion. The first portion may be positioned within the housing when the retention element is in the first position. The first portion may be configured to prevent the capsule from moving between the capsule receptacle and the aerosolization chamber. The second portion may be positioned outside of the housing when the retention element is in the first position. Advantageously, this may allow a user to pull on the second portion to move the retention element from the first position to the second position.

The first portion may be positioned within the aerosolization chamber when the retention element is in the first position.

The first portion may be configured to follow a convoluted path within the aerosolization chamber when the retention element is in the first position. For example, the convoluted path may have a sinusoidal shape or approximately a sinusoidal shape. As another example, the convoluted path may have a spiral shape or approximately a spiral shape. The first portion may be configured to follow a convoluted path from the first inlet channel to the second inlet channel. Advantageously, a convoluted path may increase the resistance to moving the retention element from the first position to the second position by introducing non-straight portions into the retention element. This may prevent the retention element from being accidentally moved from the first position to the second position. The first portion may be configured to be coiled or wound within the aerosolization chamber when retention element is in the first position. The first portion may be configured to uncoil or unwind when the retention element is moved from the first position to the second position.

The second portion may be positioned outside the inlet when the retention element is in the first position. Thus, the retention element may extend through the inlet channel when the retention element is in the first position.

The second portion may be positioned outside the outlet when the retention element is in the first position. Thus, the retention element may extend through the outlet channel when the retention element is in the first position.

The second portion may be angled with respect to the first portion to prevent the second portion from being inserted into the housing when the retention element is in the first position. For example, an angle between the first portion and the second portion may be acute. The second portion may be dimensioned to prevent the second portion from being inserted into the housing when the retention element is in the first position. Advantageously, this may prevent the second portion from accidentally entering the housing which could mean a user is unable to move the retention element from the first position to the second position.

The second portion may comprise an aperture for assisting a user in moving the retention element from the first position to the second position. The aperture may be dimensioned for insertion of a user's finger. Advantageously, providing an aperture may make it easier for a user to move the retention element from the first position to the second position. An aperture may be particularly useful where a user may not have the strength to grip the second portion between their fingers.

The retention element may be configured to substantially prevent airflow through the airflow path when the retention element is in the first position. Advantageously, this may prompt a user to move the retention element to the second position before inhaling on the inhaler. The convoluted path of the retention element may cause the retention element to prevent airflow into the aerosolization chamber.

The retention element may be configured to substantially prevent airflow from the inlet to the outlet when the retention element is in the first position. The retention element may be configured to substantially prevent airflow into the aerosolization chamber when the retention element is in the first position. The retention element may be configured to substantially prevent airflow into the inlet when the retention element is in the first position. The retention element may be configured to substantially prevent airflow exiting the outlet when the retention element is in the first position. The retention element may be configured to substantially prevent airflow through the inlet channel when the retention element is in the first position. The retention element may be configured to substantially prevent airflow through the first inlet channel when the retention element is in the first position. The retention element may be configured to substantially prevent airflow through the second inlet channel when the retention element is in the first position. The retention element may be configured to substantially prevent airflow through the outlet channel when the retention element is in the first position.

The retention element may extend through the inlet when the retention element is in the first position. Advantageously, this may obstruct the flow through the inlet and prevent airflow within the aerosolization chamber from forming a vortex. The retention element may be moved from the first position to the second position by at least partially withdrawing the retention element from the housing through the inlet. The retention element may extend from the first inlet to the second inlet when the retention element is in the first position. The retention element may be positioned within the inlet channel when the retention element is in the first position. The retention element may be positioned within the second inlet channel when the retention element is in the first position.

The retention element may be positioned in the airflow path between the aerosolization chamber and the outlet when the retention element is in the first position. That is, the retention element may be positioned within the outlet channel. The retention element may be moved from the first position to the second position by at least partially withdrawing the retention element from the housing through the outlet. Advantageously, such a retention element may allow a user to easily load the capsule receptacle with a capsule and return the retention element to the first position.

The inhaler may comprise a porous element. The porous element may be positioned in the airflow path between the aerosolization chamber and the outlet. That is, the porous element may be positioned in the outlet channel. The porous element may be configured to prevent the capsule from exiting the housing via the outlet. The composition and airflow can pass through the porous element. The porous element may span the airflow path substantially perpendicular to the direction of airflow. The porous element may be a perforated plate or grid. The porous element may be a mesh. Advantageously, the porous element may prevent the capsule from being inhaled by the user.

The retention element may abut the porous element when the retention element is in the first position. The retention element may substantially prevent airflow through the porous element when the retention element is in the first position. The retention element may comprise one or more protruding members that are configured to extend through the porous element when the retention element is in the first position. The one or more protruding members may extend into the aerosolization chamber towards the capsule receptacle opening. The retention element may comprise a plurality of protruding members. For example, the retention element may comprise four protruding members. The one or more protruding members may be spaced along a length of the capsule receptacle when the retention element is in the first position.

The retention element may comprise a retention element body. The one or more protruding members may extend from the retention element body. The retention element body may abut the porous element when the retention element is in the first position. The retention element body may extend between the porous element and the outlet when the retention element is in the first position.

The inhaler may comprise a cover. The cover may be movable between a cover position in which the outlet is at least partially covered and an uncover position in which the outlet is uncovered. The cover may at least partially cover the inlet when in the cover position.

The retention element and the cover may be a unitary component. The retention element and the cover may be integrally formed. The retention element and the cover may be integrally moulded.

The retention element may be fixed to the cover. The retention element may be fixed to the cover by an interference fit, a mechanical fit or adhesive.

The retention element may be removably couplable to the cover. The retention element may be removably couplable to the cover by an interference fit, a mechanical fit or adhesive. The retention element may be coupled to the cover when the cover is in the cover position.

Movement of the cover from the cover position to the uncover position may be configured to move the retention element from the first position to the second position. Advantageously, this reduces the number of the steps a user is required to take in order to use the inhaler to inhale the composition.

The retention element may be made from any suitable material. Examples of suitable materials include thermoplastics that are suitable for food or pharmaceutical applications, for example polypropylene, polyetheretherketone (PEEK) and polyethylene. The retention element may be made from Acrylonitrile butadiene styrene (ABS). The retention element may be made from foil. The retention element may be made from a metal foil. The retention element may be made from aluminium foil. The retention element may be made from a flexible material.

The housing may comprise a first housing portion and a second housing portion.

The first housing portion may comprise the outlet. The first housing portion may be a mouthpiece. The mouth piece may be configured to be inserted into a user's mouth. The first housing portion may be a nosepiece. The nosepiece may be configured to be inserted into a user's nose.

The second housing portion may comprise the inlet. The second housing portion may comprise the capsule receptacle. The second housing portion may comprise the aerosolization chamber. The second housing portion may comprise the rupturing element.

The first housing portion and the second housing portion may be removably couplable. That is, the coupling between the first housing portion and the second housing portion may facilitate repeated coupling and decoupling of the first housing portion and the second housing portion. Advantageously, this allows a user to access the aerosolization chamber and the capsule receptacle, thereby allowing a user to remove a used capsule from the aerosolization chamber and insert a new capsule into the capsule receptacle.

The first housing portion and the second housing portion may be couplable by mechanical keying. The first housing portion and the second housing portion may be couplable by a snap fit. The first housing portion and the second housing portion may be couplable by a screw. The first housing portion and the second housing portion may be couplable by a threaded engagement. For example, the first housing position may comprise a female thread and the second housing portion may comprise a male thread. The male thread may be threaded into the female thread. The first housing portion and the second housing portion may be magnetically couplable.

The first housing portion and the second housing portion may be configured to be rotated relative to one another to permit coupling and decoupling. The first housing portion may comprise a protrusion configured to cooperate with a hole in the second housing portion such that the first housing portion and the second housing portion must be rotated relative to one another to permit coupling and decoupling. Alternatively, the second housing portion may comprise a protrusion configured to cooperate with a hole in the first housing portion such that the first housing portion and the second housing portion must be rotated relative to one another to permit coupling and decoupling.

The housing may be formed from any suitable material or combination of materials. Examples of suitable materials include metals, alloys, plastics or composite materials containing one or more of those materials, or thermoplastics that are suitable for food or pharmaceutical applications, for example polypropylene, polyetheretherketone (PEEK) and polyethylene. Preferably, the material is light and non-brittle. The housing may be made from Acrylonitrile butadiene styrene (ABS).

The rupturing element may be a rigid element capable of piercing the capsule. The rupturing element may be a metal element. The rupturing element may be a needle. The rupturing element may be configured to move between a rupturing position and a relaxed position. In the piercing position, the rupturing element may be configured to extend into the capsule receptacle. The rupturing element may be biased to the relaxed position by a biasing element. The biasing element may be a spring. A user may press on the rupturing element to move the rupturing element from the relaxed position to the rupturing position.

The rupturing element may be operably connected to a rupturing element button. The rupturing element button may be configured to be operated to move the rupturing element from the relaxed position to the rupturing position. The rupturing element button may be a press button.

The inhaler may comprise means which, when engaged, prevents the rupturing element from being moved from the relaxed position to the rupturing position. The means may be disengaged to allow the rupturing element to be moved from the relaxed position to the rupturing position. The means may be a cover. The cover may be configured to at least partially cover the rupturing element button to prevent the rupturing element from being moved from the relaxed position to the rupturing position.

The rupturing element may be a first rupturing element. The inhaler may comprise a second rupturing element. The second rupturing element may have any of the features described in relation to the first rupturing element.

The outlet may be positioned at the proximal end of the inhaler. The aerosolization chamber may be positioned between the outlet and the capsule receptacle in a longitudinal direction.

The aerosolization chamber may be cylindrical.

The aerosolization may have height dimension in the longitudinal direction. The aerosolization chamber may have a height between 20 millimetres and 50 millimetres, between 20 millimetres and 40 millimetres or between 20 millimetres and 30 millimetres. The aerosolization chamber may have a height of 25 millimetres.

The aerosolization chamber may have a diameter (or width) dimension in direction transverse to the longitudinal direction. The aerosolization chamber may have a diameter (or width) between 30 millimetres and 60 millimetres, between 40 millimetres and 60 millimetres, between 50 millimetres and 60 millimetres. The aerosolization chamber may have a diameter (or width) of 55 millimetres.

The capsule receptacle may have height dimension in the longitudinal direction. The capsule receptacle may have a height of between 10 millimetres and 20 millimetres. The capsule receptacle may have a height of about 10 millimetres, 11 millimetres, 12 millimetres, 13 millimetres, 14 millimetres, 15 millimetres, 16 millimetres, 17 millimetres, 18 millimetres, 19 millimetres or 10 millimetres.

The capsule receptacle may have a width dimension in direction transverse to the longitudinal direction. The capsule receptacle may have a width of between 10 millimetres and 20 millimetres. The capsule receptacle may have a width of about 10 millimetres, 11 millimetres, 12 millimetres, 13 millimetres, 14 millimetres, 15 millimetres, 16 millimetres, 17 millimetres, 18 millimetres, 19 millimetres or 10 millimetres.

The capsule receptacle may have a length dimension in direction transverse to the longitudinal direction and the width dimension. The capsule receptacle may have a length between 30 millimetres and 60 millimetres, between 40 millimetres and 60 millimetres, between 50 millimetres and 60 millimetres. The capsule receptacle may have a length of 55 millimetres.

The inhaler may comprise a capsule positioned within the capsule receptacle. The capsule may comprise the composition.

The capsule receptacle may be elongate. Thus, the length of the capsule receptacle may be greater than a width of the capsule receptacle. The capsule receptacle may be dimensioned to receive a capsule from size 0 to size 4. For example, size 0, size 1, size 2, size 3 or size 4.

The capsule may comprise a capsule shell for encapsulating the composition. The capsule may be any suitable pharmaceutical capsule, such as a hard-shelled capsule. The capsule shell may be manufactured from gelling agents such as gelatin and/or polysaccharides. The capsule shell may be formed from hydroxypropyl methyl cellulose (HPMC). The capsule shell may comprise plasticizers, such as glycerin or sorbitol.

The composition may be in the form of a dry powder suitable for dry powder inhalation. The capsule may be any suitable size. The capsule may be a size 1 to size 4 capsule.

Preferably, the composition comprises a pharmaceutically active ingredient. The pharmaceutical active ingredient may be capable of treating or preventing a thromboembolic event. The pharmaceutically active ingredient may be an antiplatelet drug. For example, the pharmaceutically active ingredient may be a non-steroidal anti-inflammatory drug (NSAID). Preferably, the pharmaceutically active ingredient is a salicylate (a salt or ester of salicylic acid), most preferably acetylsalicylic acid or a pharmaceutically acceptable salt thereof.

The pharmaceutically active ingredient may be another type of NSAID. For example, the pharmaceutically active ingredient may Celecoxib (Celebrex), Dexdetoprofen (Keral), Diclofenac (Voltaren, Cataflam, Voltaren-XR), Diflunisal (Dolobid), Etodolac (Lodine, Lodine XL), Etoricoxib (Algix), Fenoprofen (Fenopron, Nalfron), Firocoxib (Equioxx, Previcox), Flurbiprofen (Urbifen, Ansaid, Flurwood, Proben), Ibuprofen (Advil, Brufen, Motrin, Nurofen, Medipren, Nuprin), Indomethacin (Indocin, Indocin SR, Indocin IV), Ketoprofen (Actron, Orudis, Oruvail, Ketoflam), Ketorolac (Toradol, Sprix, Toradol IV/1M, Toradol IM), Licofelone (under development), Lomoxicam (Xefo), Loxoprofen (Loxonin, Loxomac, Oχeηo), Lumiracoxib (Prexige), Meclofenamic acid (Meclomen), Mefenamic acid (Ponstel), Meloxicam (Movalis, Mel ox, Recoxa, Mobic), Nabumetone (Relafen), Naproxen (Aleve, Anaprox, Midol Extended Relief, Naprosyn, Naprelan), Nimesulide (Sulide, Nimalox, Mesulid), Oxaporozin (Daypro, Dayrun, Duraprox), Parecoxib (Dynastat), Piroxicam (Feldene), Rofecoxib (Vioxx, Ceoxx, Ceeoxx), Salsalate (Mono-Gesic, Salflex, Disalcid, Salsitab), Sulindac (Clinoril), Tenoxicam (Mobi flex), Tolfenamic acid (Clotam Rapid, Tufnil), or Valdecoxib (Bextra).

The pharmaceutically active ingredient may be an alternative to an NSAID. Such alternatives include P2Y12 inhibitors. Examples of P2Y12 inhibitors include Plavix (clopidogrel), ticlopidine, ticagrelor, prasugrel, and cangrelor. Other pharmaceutically active ingredients may include COX-2 inhibitors, and Nattokinase (an enzyme (EC 3.4.21.62, extracted and purified from a Japanese food called natto)).

The composition may comprise both acetylsalicylic acid, or a pharmaceutically acceptable salt thereof, and a P2Y12 inhibitor.

Preferably, the pharmaceutically active ingredient is at least 80% by weight, preferably at least 90% by weight, more preferably at least 95% by weight of the composition. For example, at least 95% by weight of the composition in the capsule may be acetylsalicylic acid or a pharmaceutically acceptable salt thereof.

The composition preferably comprises a pharmaceutically acceptable excipient. For example, the excipient may be an anti-aggregation excipient, an anti-adherent or a lubricant. The pharmaceutically acceptable excipient may be magnesium stearate. The magnesium stearate may be present in an amount of 1% or less, by weight, of the composition, such as 0.05 to 1%, by weight, of the composition. For example the magnesium stearate may be present in an amount of about 0.5%, by weight.

The amount, or dose, of the pharmaceutically active ingredient (preferably acetylsalicylic acid or salt thereof) in the capsule is preferably 100 mg or less, more preferably 75 mg or less, even more preferably 60 mg or less.

The amount, or dose, of the pharmaceutically active ingredient (preferably acetylsalicylic acid or salt thereof) in the capsule may be at least 5 mg, preferably at least 20 mg, more preferably at least 30 mg, even more preferably at least 40 mg.

The amount, or dose, of the pharmaceutically active ingredient (preferably acetylsalicylic acid or salt thereof) in the capsule may be 5-300 mg, preferably 20-100 mg, more preferably 30-75 mg, even more preferably 40-60 mg. For example, there may be about 50 mg of acetylsalicylic acid or salt thereof in the capsule.

A user may require multiple doses of the pharmaceutically active ingredient (preferably acetylsalicylic acid or salt thereof) when treating a condition, such as treating or preventing a thromboembolic event. The inhaler may include a single capsule with a pre-determined dose of the active ingredient. In order to administer multiple doses, the user may require multiple inhalers, with each inhaler providing a single dose.

For example, the inhaler may include a single capsule and the capsule may include less than 100 mg (preferably about 50 mg) of acetylsalicylic acid or salt thereof in dry powder form. When treating or preventing a thromboembolic event, the user may use two inhalers, each inhaler providing a single dose, to provide a total of two doses.

The composition is preferably a respirable dry powder consisting of respirable dry particles which are suitable for delivery to the respiratory tract (e.g., pulmonary delivery) in a user by inhalation.

The particles may have a mass median aerodynamic diameter (MMAD) of 10 µm or less, preferably 5 µm or less (such as 0.5-5 µm).

The particles may have a volumetric median geometric diameter (VMGD) as measured by HELOS/RODOS at 1.0 bar, of 10 µm or less, preferably 5 µm or less (such as 0.5-5 µm).

The particles may have a geometric diameter distribution, or an aerodynamic diameter distribution, where the particles exhibit a DV90 less than 10 µm, a DV50 less than 4 pm, and a DV10 less than 1 µm; or preferably a DV90 less than 6 µm, a DV50 less than 3 µm, and a DV10 less than 1 µm.

The diameter of the respirable dry particles, for example, their VMGD, can be measured using an electrical zone sensing instrument such as a Multisizer lie, (Coulter Electronic, Luton, Beds, England), or a laser diffraction instrument such as a HELOS system (Sympatec, Princeton, WO 2016/019253).

Experimentally, aerodynamic diameter can be determined using time of flight (TOF) measurements. For example, an instrument such as the Model 3225 Aerosizer DSP Particle Size Analyzer (Amherst Process Instrument, Inc., Amherst, MA) can be used to measure aerodynamic diameter. The Aerosizer measures the time taken for individual respirable dry particles to pass 10 between two fixed laser beams. Aerodynamic diameter also can be experimentally determined directly using conventional gravitational settling methods, in which the time required for a sample of respirable dry particles to settle a certain distance is measured. Indirect methods for measuring the mass median aerodynamic diameter include the Andersen Cascade Impactor (ACI) and the multi-stage liquid impinger (MSFI) methods. Another method of measuring the aerodynamic diameter is with a Next Generation Impactor (NGI). The NGI operates on similar principles of inertial impaction as the ACI. The NGI consists of seven stages and can be calibrated at flow rates of 30, 60, and 100 FPM. In contrast to the ACI, for which the impactor stages are stacked, the stages of the NGI are all in one plane. Collection cups are used to collect the particles below each stage of 20 the NGI. U.S. Patent No. 8,614,255.

The inhaler may be disposed within a sealed container. That is, the inhaler may be sealed within the sealed container. The container may be hermetically sealed. The container may be tamper-evident. That is, the container may irreversibly change when the container is tampered with, such as when the container is unsealed. For example, the container may irreversibly rip or tear when unsealed. The inhaler may comprise a capsule positioned within the capsule receptacle. The retention element may be in the first position.

According to the present disclosure, there is provided an inhaler hermetically sealed within a container. The inhaler comprises a housing defining an inlet, an outlet and an airflow path extending from the inlet to the outlet. The inhaler comprises a capsule within the housing. The inhaler may be configured to rupture the capsule to allow the composition to be inhaled by a user drawing on the outlet. The inhaler hermetically sealed within the container may have any of the features of an inhaler described herein.

According to the present disclosure, there is provided a method of administering a composition with the inhaler as described herein. The inhaler comprising a capsule as described herein positioned within the capsule receptacle. The capsule comprising a composition as described herein. The method comprises rupturing the capsule with the rupturing element; moving the retention element from the first position to the second position; and drawing on the outlet to inhale the composition. The composition may be any composition described herein.

According to present disclosure, there is provided a composition. The composition comprises an antiplatelet drug for use in a method of treating, preventing or ameliorating a thromboembolic event in a patient. The composition is administered to the patient using the inhaler described herein. The inhaler comprising a capsule as described herein positioned within the capsule receptacle. The capsule comprising a composition as described herein. The composition being administered by: rupturing the capsule with the rupturing element; moving the retention element from the first position to the second position; and drawing on the outlet to inhale the composition.

According to the present disclosure, there is provided a method of treating, preventing or ameliorating a thromboembolic event in a patient. The method comprises administering a composition comprising an antiplatelet drug to a patient in need thereof. The composition is administered to the patient using the inhaler described herein. The inhaler comprising a capsule as described herein positioned within the capsule receptacle. The capsule comprising a composition as described herein. The composition being administered by: rupturing the capsule with the rupturing element; moving the retention element from the first position to the second position; and drawing on the outlet to inhale the composition.

According to present disclosure, there is provided a use of a composition comprising an antiplatelet drug in the manufacture of a medicament for treating, preventing or ameliorating a thromboembolic event in a patient. The composition is administered to the patient using the inhaler described herein. The inhaler comprising a capsule as described herein positioned within the capsule receptacle. The capsule comprising a composition as described herein. The composition being administered by: rupturing the capsule with the rupturing element; moving the retention element from the first position to the second position; and drawing on the outlet to inhale the composition.

The antiplatelet drug for use in treating, preventing or ameliorating a thromboembolic event may be an antiplatelet drug as described herein, such as an NSAID or a P2Y12 inhibitor. Preferably, the antiplatelet drug is acetylsalicylic acid or a pharmaceutically acceptable salt thereof.

The invention is defined in the claims. However, below there is provided a non-exhaustive list of non-limiting examples. Any one or more of the features of these examples may be combined with any one or more features of another example, embodiment, or aspect described herein.
Ex1. An inhaler comprising:
   a housing defining an inlet, an outlet, and an airflow path extending from the inlet to the outlet;
   a capsule receptacle for receiving a capsule comprising a composition for inhalation;
   a rupturing element for rupturing the capsule received in the capsule receptacle;
   an aerosolization chamber for allowing the composition to be entrained in airflow within the airflow path; and
   a retention element movable between a first position in which the capsule is prevented from moving from the capsule receptacle to the aerosolization chamber, and a second position in which the capsule is movable from the capsule receptacle to the aerosolization chamber.
Ex2. An inhaler according to Ex1, wherein the inhaler is a dry powder inhaler.
Ex3. An inhaler according to Ex1 or Ex2, wherein the retention element is separated from the housing when the retention element is in the second position.
Ex4. An inhaler according to any preceding example comprising an actuating means configured to move the retention element from the first position to the second position.
Ex5. An inhaler according to Ex4, wherein the actuating means comprises a mechanical actuating means.
Ex6. An inhaler according to Ex4 or Ex5, wherein the actuating means comprise a user-operable button operable to activate the actuating means.
Ex7. An inhaler according to Ex6, wherein the user-operable button is positioned at an external surface of the housing.
Ex8. An inhaler according to Ex6 or Ex7, wherein the user-operable button is a press button.
Ex9. An inhaler according to Ex6 or Ex7, wherein the user-operable button is a slide button.
Ex10. An inhaler according to any preceding example, wherein the retention element is configured to move from the first position to the second position when a user inhales on the inhaler.
Ex11. An inhaler according to any preceding example, wherein the retention element is configured such that airflow within the airflow path causes the retention element to move from the first position to the second position.
Ex12. An inhaler according to any preceding example, wherein the retention element is configured to move from the first position to the second position when the rate of airflow within the airflow path is above a rate of airflow threshold.
Ex13. An inhaler according to any preceding example, wherein the inhaler is configured to prevent movement of the retention element from the first position to the second position until the capsule has been ruptured.
Ex14. An inhaler according to any preceding example, wherein movement of the retention element from the first position to second position is configured to cause the rupturing element to rupture the capsule.
Ex15. An inhaler according to any preceding example, wherein movement of the retention element from the first position to the second position is configured to cause the capsule to move from the capsule receptacle to the aerosolization chamber.
Ex16. An inhaler according to any preceding example, wherein movement of the retention element from the first position to the second position is configured to cause the capsule to move from the capsule receptacle to the aerosolization chamber, and subsequently prevent the capsule from moving from the aerosolization chamber to the capsule receptacle.
Ex17. An inhaler according to any preceding example, wherein the retention element is configured to contact the capsule within the capsule receptacle when the retention element is in the first position.
Ex18. An inhaler according to any preceding example, wherein the retention element is configured to substantially prevent movement of the capsule with respect to the capsule receptacle when the retention element is in the first position.
Ex19. An inhaler according to any preceding example, wherein the retention element is configured to substantially prevent movement of the capsule with respect to the capsule receptacle when the retention element is in the first position by urging the capsule against a wall of the capsule receptacle.
Ex20. An inhaler according to any preceding example, wherein the capsule receptacle comprises a capsule receptacle opening for allowing a capsule to move between the capsule receptacle and the aerosolization chamber.
Ex21. An inhaler according to Ex20, wherein the retention element is configured to at least partially cover the capsule receptacle opening when the retention element is the first position.
Ex22. An inhaler according to Ex20 or Ex21, wherein the retention element is configured to substantially prevent airflow through the airflow path from entering into the capsule receptacle when the retention element is in the first position.
Ex23. An inhaler according to any preceding example, wherein the retention element comprises a first portion and a second portion, wherein the first portion is positioned within the housing when the retention element is in the first position and the second portion is positioned outside of the housing when the retention element is in the first position.
Ex24. An inhaler according to Ex23, wherein the first portion is positioned within the aerosolization chamber when the retention element is in the first position.
Ex25. An inhaler according to Ex24, wherein the first portion is configured to follow a convoluted path within the aerosolization chamber when the retention element is in the first position.
Ex26. An inhaler according to Ex25, wherein the convoluted path has a sinusoidal shape or a spiral shape.
Ex27. An inhaler according to any one of Ex23 to Ex26, wherein the first portion is configured to be coiled or wound within the aerosolization chamber when retention element is in the first position.
Ex28. An inhaler according to any one of Ex23 to Ex27, wherein the first portion is configured to uncoil or unwind when the retention element is moved from the first position to the second position.
Ex29. An inhaler according to any one of Ex23 to Ex28, wherein the second portion is angled with respect to the first portion to prevent the second portion from being inserted into the housing when the retention element is in the first position.
Ex30. An inhaler according to Ex29, wherein an angle between the first portion and the second portion is acute.
Ex31. An inhaler according to Ex23 to Ex30, wherein the second portion comprises an aperture for assisting a user in moving the retention element from the first position to the second position.
Ex32. An inhaler according to any preceding example, wherein the retention element is configured to substantially prevent airflow through the airflow path when the retention element is in the first position.
Ex33. An inhaler according to any preceding example, wherein the retention element is configured to substantially prevent airflow into the inlet.
Ex34. An inhaler according to any preceding example, wherein the retention element extends through the inlet when the retention element is in the first position.
Ex35. An inhaler according to any preceding example, wherein the retention element is positioned in the airflow path between the aerosolization chamber and the outlet when the retention element is in the first position.
Ex36. An inhaler according to any one of Ex1 to Ex35, wherein the retention element is moved from the first position to the second position by at least partially withdrawing the retention element from the housing through the inlet.
Ex37. An inhaler according to any one of Ex1 to Ex35, wherein the retention element is moved from the first position to the second position by at least partially withdrawing the retention element from the housing through the outlet.
Ex38. An inhaler according to any preceding example comprising a porous element positioned in the airflow path between the aerosolization chamber and the outlet.
Ex39. An inhaler according to Ex38, wherein the porous element is configured to prevent the capsule from exiting the housing via the outlet.
Ex40. An inhaler according to Ex39, wherein the porous element is a mesh.
Ex41. An inhaler according to any one of Ex38 to Ex40, wherein the retention element abuts the porous element when the retention element is in the first position.
Ex42. An inhaler according to any one of Ex38 to Ex41, wherein the retention element substantially prevents airflow through the porous element when the retention element is in the first position.
Ex43. An inhaler according to any one of Ex38 to Ex42, wherein the retention element comprises one or more protruding members that are configured to extend through the porous element when the retention element is in the first position.
Ex44. An inhaler according to any preceding example comprising a cover, wherein the cover is movable between a cover position in which the outlet is at least partially covered and an uncover position in which the outlet is uncovered.
Ex45. An inhaler according to Ex44, wherein the retention element is fixed to the cover.
Ex46. An inhaler according to Ex44 or Ex45, wherein movement of the cover from the cover position to the uncover position is configured to move the retention element from the first position to the second position.
Ex47. An inhaler according to any preceding example, wherein the housing comprises a first housing portion and a second housing portion, wherein the first housing portion may comprises the outlet and the second housing portion comprises the inlet, the capsule receptacle, the aerosolization chamber and the rupturing element.
Ex48. An inhaler according to Ex47, wherein the first housing portion and the second housing portion are removably couplable.
Ex49. An inhaler according to any preceding example comprising a capsule positioned within the capsule receptacle, the capsule comprising a composition.
Ex50. An inhaler according to Ex49, wherein the composition is in the form of a dry powder.
Ex51. An inhaler according to Ex49 or Ex50, wherein the composition comprises a pharmaceutically active ingredient.
Ex52. An inhaler according to Ex51, wherein the pharmaceutical active ingredient is capable of treating or preventing a thromboembolic event, or is an anti-platelet drug.
Ex53. An inhaler according to Ex51 or Ex52, wherein the pharmaceutically active ingredient is a non-steroidal anti-inflammatory drug (NSAID).
Ex54. An inhaler according to any one of Ex51 to Ex53, wherein the pharmaceutically active ingredient is a salicylate (a salt or ester of salicylic acid), preferably acetylsalicylic acid or a pharmaceutically acceptable salt thereof.
Ex55. An inhaler according to Ex51 or Ex52, wherein the pharmaceutically active ingredient is a P2Y12 inhibitor.
Ex56. A method of administering a composition with the inhaler of any one of Ex1 to Ex55 having a capsule positioned within the capsule receptacle and the capsule comprising a composition, the method comprising:
   rupturing the capsule with the rupturing element;
   moving the retention element from the first position to the second position; and drawing on the outlet to inhale the composition.
Ex57. A composition comprising an antiplatelet drug for use in a method of treating, preventing or ameliorating a thromboembolic event in a patient, wherein the composition is administered to the patient using the inhaler of any one of Ex1 to Ex55 having a capsule positioned within the capsule receptacle and the capsule comprising the composition, by:
   rupturing the capsule with the rupturing element;
   moving the retention element from the first position to the second position; and drawing on the outlet to inhale the composition.
Ex58. A composition for the use according to Ex57 wherein the antiplatelet drug is an NSAID or a P2Y12 inhibitor.
Ex59. A composition for the use according to EX58, wherein the antiplatelet drug is acetylsalicylic acid or a pharmaceutically acceptable salt thereof.
Ex60. An inhaler hermetically sealed within a container, the inhaler comprising:
   a housing comprising an inlet, an outlet and an airflow path extending from the inlet to the outlet; and
   a capsule within the housing,
   wherein the inhaler is configured to rupture the capsule to allow the composition to be inhaled by a user drawing on the outlet.
Ex61. A method of administering a composition with the inhaler of Ex60, the method comprising:
   removing the inhaler from the container;
   rupturing the capsule; and
   drawing on the outlet to inhale the composition.
Ex62. A composition comprising acetylsalicylic acid or a pharmaceutically acceptable salt thereof for use in a method of treating, preventing or ameliorating a thromboembolic event in a patient, wherein the composition is administered to the patient using the inhaler of Ex60, by:
   removing the inhaler from the container;
   rupturing the capsule; and
   drawing on the outlet to inhale the composition.

Examples will now be further described with reference to the figures in which:
Figure 1A shows a cross-sectional view of an inhaler having a retention element according to a first example of the present disclosure;
Figure 1B shows a perspective cross-sectional view of the inhaler of Figure 1A while in use;
Figure 2 shows a cross-sectional view of an inhaler having a retention element according to a second example of the present disclosure;
Figure 3A shows a perspective view of a first housing portion of an inhaler having a retention element according to a third example of the present disclosure;
Figure 3B shows a top view of the first housing portion of Figure 3A;
Figure 3C shows a cross-sectional side view of the first housing portion of Figure 3A and 3B cut along line A-A;
Figure 4A shows a perspective view of a first housing portion of an inhaler having a retention element according to a fourth example of the present disclosure;
Figure 4B shows a top view of the first housing portion of Figure 4A; and
Figure 4C shows a cross-sectional side view of the first housing portion of Figure 4A and 4B cut along the line B-B.

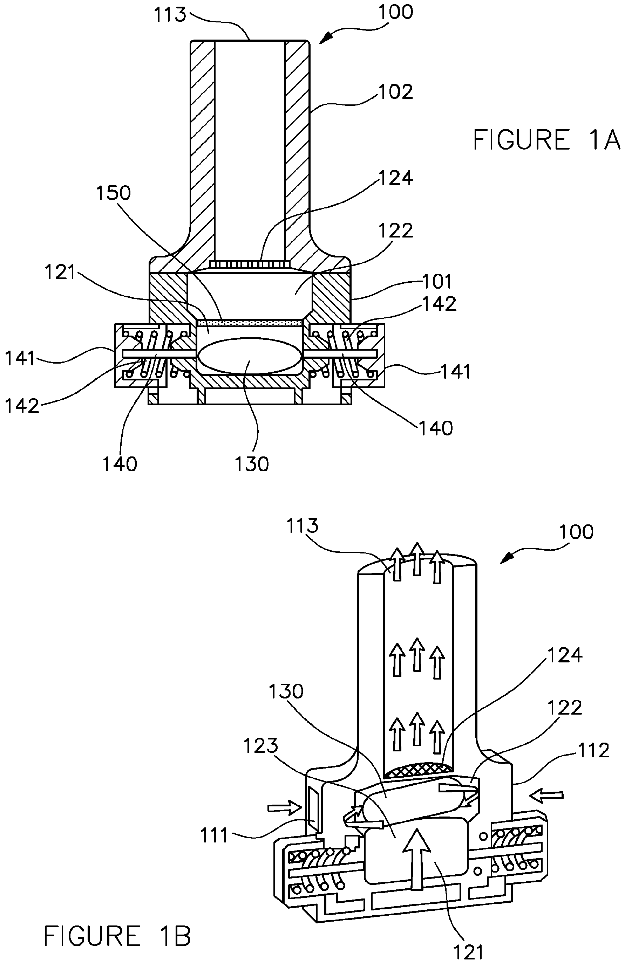

Figure 1A and 1B show an inhaler 100 having a retention element 150 according to a first example of the present disclosure. The inhaler 100 comprises a housing defining a first inlet 111, a second inlet 112, an outlet 113, and an airflow path extending from the first inlet 111 and second inlet 112 to the outlet 113. The housing is formed from a first housing portion 101 and a second housing portion 102. Further aspects of the first housing portion 101 and the second housing portion 102 will be shown in other Figures.

The first housing portion 101 comprises a capsule receptacle 121 for receiving a capsule 130. The capsule 130 comprises a composition for inhalation. The first housing portion 101 also comprises first and second rupturing elements 140 for rupturing the capsule 130 received in the capsule receptacle 121. The rupturing elements 140 are needles that create a hole in each end of the capsule when actuated. The rupturing elements 140 are biased to a relaxed position by springs 142. The rupturing elements 140 are shown in the relaxed position in Figures 1A and 1B. The rupturing elements 140 and the springs 142 are operably connected to rupturing element buttons 141 to facilitate actuation of the rupturing elements 140. To actuate the rupturing elements 140, a user presses on the rupturing element buttons 141 in a direction towards the capsule receptacle 121. This causes the rupturing elements 140 to extend into the capsule receptacle 121 and rupture the capsule 130. The rupturing elements 140 may be individually actuated. The first housing portion 101 also comprises an aerosolization chamber 122 for allowing the composition within a pierced capsule to be entrained in airflow within the airflow path.

The inhaler 100 further comprises a retention element 150 that is movable between a first position in which the capsule 130 is prevented from moving from the capsule receptacle 121 to the aerosolization chamber 122, and a second position in which the capsule 130 is movable from the capsule receptacle 121 to the aerosolization chamber 122. In Figure 1A, the retention element 150 is in the first position in which the retention element 150 acts a physical barrier to prevent the capsule 130 from moving from the capsule receptacle 121 to the aerosolization chamber 122 by covering an opening 123 between the capsule receptacle 121 and the aerosolization chamber 122. In the second position, the retention element 130 is retracted and does not cover the opening between the capsule receptacle 121 and the aerosolization chamber 122. In Figure 1A and 1B, the retention element is moved from the first position to the second position by a user pressing on a user-operable button (not shown).

The second housing portion 102 comprises an outlet 113. A user may draw on the outlet 113 to inhale the composition that is within the capsule 130. The first housing portion 101 and the second housing portion 102 are removably couplable. This provides access to the capsule receptacle 121 and the aerosolization chamber 122.

Figure 1B shows the inhaler 100 of Figure 1A during use. In Figure 1B, the capsule 130 has been ruptured with the rupturing elements 140. Furthermore, the retention element 150 has been moved from the first position to the second position such that the opening 123 between the capsule receptacle 121 and the aerosolization chamber 122 is uncovered. This allows the capsule 130 to move from the capsule receptacle 121 to the aerosolization chamber 122.

The airflow path extends between the inlets 111, 112 and the outlet 113 through the aerosolization chamber 122. The aerosolization chamber 122 has a substantially circular wall. The inlet channel between the first inlet 111 and the aerosolization chamber 122, and the inlet channel between the second inlet 112 and the aerosolization chamber 122 are arranged to cause airflow to enter the aerosolization chamber 122 in a direction tangential to the circular wall of the aerosolization chamber 122. Thus, as a user draws on the outlet 113 of the inhaler, air is drawn through the first inlet 111 and the second inlet 112 and forms a vortex within the aerosolization chamber 122. The vortexed airflow causes the capsule 130 to move from the capsule receptacle 121 to the aerosolization chamber 122. Once the capsule 130 is within the aerosolization chamber 122, the vortexed airflow causes the capsule 130 to rotate and release the composition into the airflow such that the composition is entrained within the airflow and delivered to the user via the outlet 113. The second housing portion 102 includes a mesh 124 that allows the composition to pass through it but does not allow the capsule 130 to pass through it.

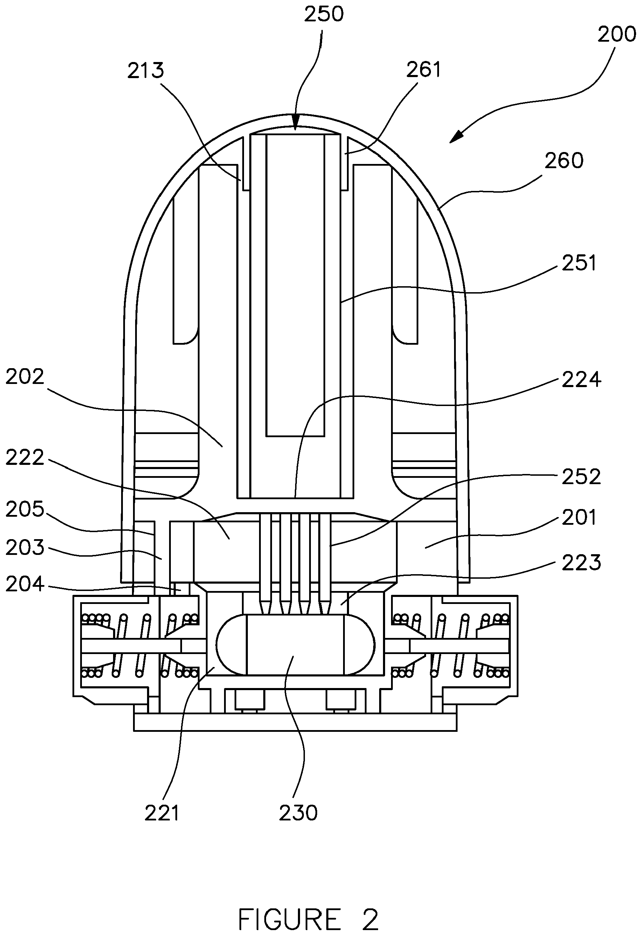

Figure 2 shows an inhaler 200 having a retention element 250 according to a second example of the present disclosure. The components of the first housing portion 201 and second housing portion 202 are the same as the components of the first housing portion 101 and second housing portion 102 in Figures 1A and 1B.

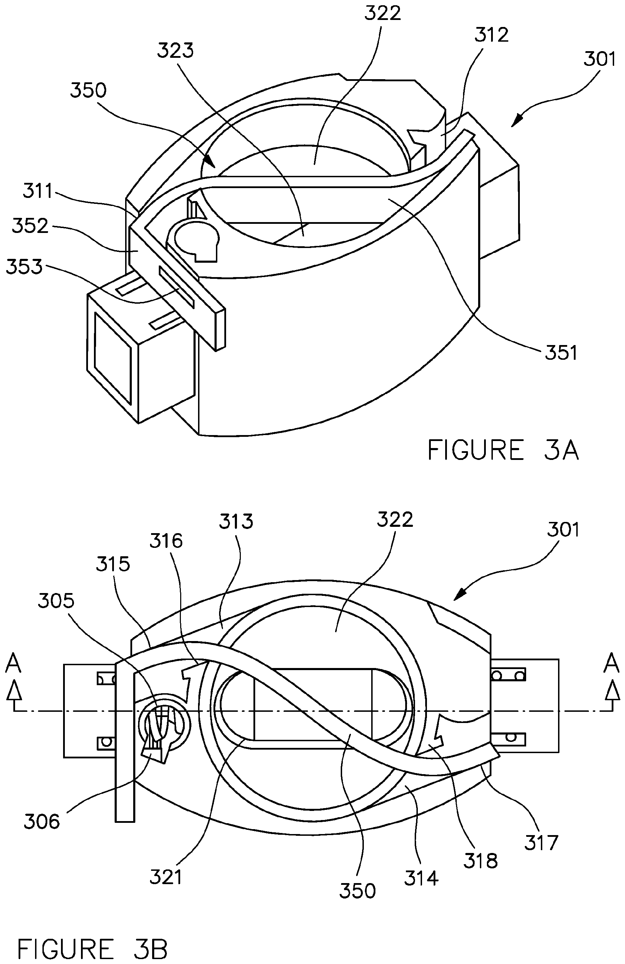

Figure 2 shows additional features of the first housing portion 201 and second housing portion 202 which are not shown in Figure 1A or 1B. In particular, it can be seen that the first housing portion 201 comprises a hole 205 that is configured to cooperate with a peg 203 extending from the second housing portion 202 to allow the first housing portion 201 and the second housing portion 202 to be removably couplable. The peg 203 has a radial protrusion 204 that cooperates with a recess in the hole 205 that requires the first housing portion 201 and the second housing portion 202 to be rotated relative to one another to permit coupling and decoupling. Figure 3B depicts the hole 305 having the recess 306.

The inhaler 200 also comprises a cover 260. In Figure 2, the cover 260 is depicted in a cover position in which the first inlet 111, the second inlet 112 and the outlet 213 are covered. In the cover position, air cannot flow through the airflow path, however, a user may still actuate the rupturing elements to rupture the capsule 230 within the capsule receptacle 221.

The retention element 250 comprises a retention element body 251 and a plurality of protruding members 252 that extend from the retention element body 251. The retention element 250 is depicted in the first position in Figure 2. In the first position, the retention element body 251 is positioned within the airflow path and abuts the mesh 224. The retention element body 251 extends between the mesh 224 and the outlet 213. The plurality of protruding members 252 extend through the mesh 224 and the aerosolization chamber 222 towards the capsule 230 within the capsule receptacle 221.

The retention element body 251 is positioned within an cavity of the cover 260. The cavity has a circular wall 261. The retention element body 251 forms an interference fit with the circular wall 261 such that the retention element 250 is movably connected to the cover 260. When the cover 260 is removed from the inhaler 200, the retention element 250 is separated from the housing through the outlet 213. Thus, removal of the cover 260 causes the retention element 250 to move from the first position to the second position in which the capsule 230 can move from the capsule receptacle 221 to the aerosolization chamber 222. In some other embodiments, the retention element 250 and the cover 260 may not be movably connected. In these embodiments, the cover 260 may first be removed from the inhaler 200 to access the retention element 250. The retention element 250 may then be moved from the first position to the second position by pulling on an end of the retention element 250 that is adjacent the outlet 213.

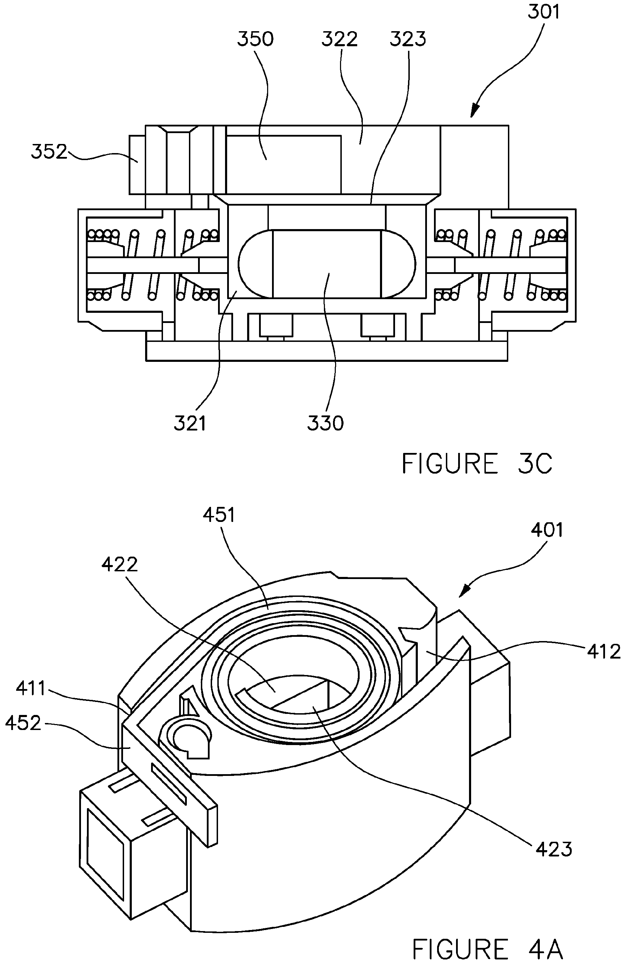

Figures 3A, 3B and 3C show three different views of a retention element 350, according to a third example of the present disclosure, in the first position. For simplicity, only the first housing portion 301 is shown, however, the first housing portion 301 is identical to the first housing portion 101 shown in Figures 1A and 1B. Thus, the first housing portion 301 in Figures 3A, 3B and 3C may be used with the second housing portion 102 shown in Figures 1A and 1B.

The retention element 350 is in the form of a strip having a generally rectangular cross-section and is made from a flexible material which allows the retention element 350 to bend as it is moved from the first position to the second position.

The first housing portion 301 comprises a first inlet channel 313 extending between the first inlet 311 and the aerosolization chamber 322. The first inlet channel 313 comprises a first wall 315 disposed opposite a second wall 316. The first housing portion 301 comprises a second inlet channel 314 extending between the second inlet 312 and the aerosolization chamber 322. The second inlet channel 314 comprises a first wall 317 disposed opposite a second wall 318.

In the first position, the retention element 350 has a first portion 351 that is positioned within the first housing portion 301. The retention element 350 extends through both the first inlet channel 313 and the second inlet channel 314. The first portion 351 of the retention element 350 extends through the first inlet 311 and the second inlet 312 along a sinusoidal path. Thus, the section of the retention element 350 that is positioned within the first inlet channel 313, and the section of the retention element 350 that is positioned with the second inlet channel 314, are curved. As a result, the section of the retention element 350 that is positioned within the first inlet channel 313 contacts both the first wall 315 and the second wall 316 of the first inlet channel 313. This prevents airflow through the first inlet 311. Similarly, the section of the retention element 350 that is positioned within the second inlet channel 314 contacts both the first wall 317 and the second wall 318 of the second inlet channel 314. This prevents airflow through the second inlet 312. The first portion 351 of the retention element 350 extends across the aerosolization chamber 322 and partially covers the opening 323 between the capsule receptacle 321 and the aerosolization chamber 322. This prevents the capsule 330 from moving from the capsule receptacle 321 to the aerosolization chamber 322.

In the first position, the retention element 350 has a second portion 352 that is positioned outside of the first housing portion 301. To move the retention element 350 from the first position to the second position, a user pulls on the second portion 352 of the retention element 350 so as to withdraw the retention element 350 from the first housing portion 301 through the first inlet 311. The retention element 350 is able to bend as it is withdrawn from the first housing portion 301 because it is made from a flexible material. In the second position, the retention element 350 is separated from the first housing portion 301. The second portion 352 comprises an aperture 353 to assist a user in moving the retention element 350 from the first position to the second position. The second portion 352 of the retention element 350 is angled with respect to the first portion 351 of the retention element to prevent it from being accidently inserted or retracted into the first housing portion 301.

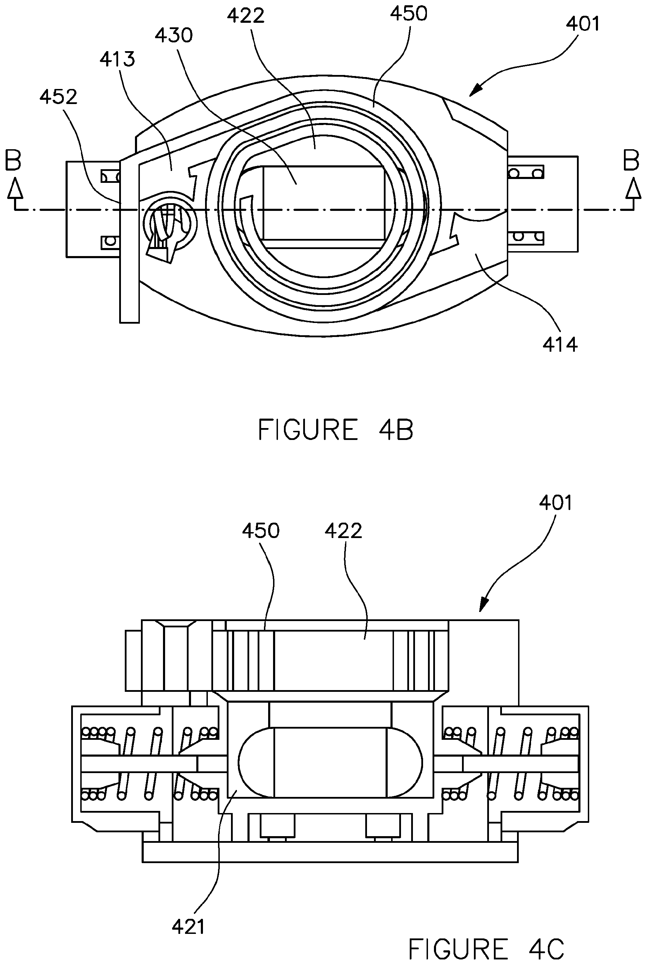

Figure 4A, 4B and 4C show three different views of a retention element 450, according to a fourth example of the present disclosure, in the first position. For simplicity, only the first housing portion 401 is shown, however, the first housing portion 401 is identical to the first housing portion 101 shown in Figures 1A and 1B. Thus, the first housing portion 401 in Figures 4A, 4B and 4C may be used with the second housing portion 102 shown in Figures 1A and 1B.

The retention element 450 is in the form of a strip having a generally rectangular cross-section and is made from a flexible material that allows the retention element 450 to bend as it is moved from the first position to the second position.

In the first position, the retention element 450 has a first portion 451 that is positioned within the first housing portion 401. The first portion 451 of the retention element 450 is coiled within the aerosolization chamber 422. The first portion 451 of the retention element 450 partially covers the opening 423 between the capsule receptacle 421 and the aerosolization chamber 422. This prevents the capsule 430 from moving from the capsule receptacle 421 to the aerosolization chamber 422. The retention element 450 extends through the first inlet channel 413 but not the second inlet channel 414. Nevertheless, it can be seen that the first portion 451 of the retention element 450 is coiled such that it prevents airflow within the second inlet channel 414 from entering into the aerosolization chamber 422. It can be seen that the second portion 452 of the retention element 450 prevents air from flowing into the first inlet 411.

To move the retention element 450 from the first position to the second position, a user pulls on the second portion 452 of the retention element 450 so as to withdraw the retention element 450 from the first housing portion 401 through the first inlet 411. As the retention element 450 is withdrawn from the first housing portion 401, the first portion 451 of the retention element 450 uncoils. In the second position, the retention element 450 is separated from the first housing portion 401. The second portion 452 comprises an aperture to assist a user in moving the retention element 450 from the first position to the second position. The second portion 452 of the retention element 450 is angled with respect to the first portion 451 of the retention element 450 to prevent it from being accidently inserted or retracted into the first housing portion 401.

For the purpose of the present description and of the appended claims, except where otherwise indicated, all numbers expressing amounts, quantities, percentages, and so forth, are to be understood as being modified in all instances by the term "about". Also, all ranges include the maximum and minimum points disclosed and include any intermediate ranges therein, which may or may not be specifically enumerated herein. In this context, therefore, a number A is understood as A ± 5% of A. Within this context, a number A may be considered to include numerical values that are within general standard error for the measurement of the property that the number A modifies. The number A, in some instances as used in the appended claims, may deviate by the percentages enumerated above provided that the amount by which A deviates does not materially affect the basic and novel characteristic(s) of the claimed invention. Also, all ranges include the maximum and minimum points disclosed and include any intermediate ranges therein, which may or may not be specifically enumerated herein.

## Claims

1. An inhaler comprising:
a housing defining an inlet, an outlet, and an airflow path extending from the inlet to the outlet;
a capsule receptacle for receiving a capsule comprising a composition for inhalation;
a rupturing element for rupturing the capsule received in the capsule receptacle;
an aerosolization chamber for allowing the composition to be entrained in airflow within the airflow path; and
a retention element movable between a first position in which the capsule is prevented from moving from the capsule receptacle to the aerosolization chamber, and a second position in which the capsule is movable from the capsule receptacle to the aerosolization chamber.

2. An inhaler according to claim 1, wherein the retention element is separated from the housing when the retention element is in the second position.

3. An inhaler according to claim 1 or 2 comprising an actuating means configured to move the retention element from the first position to the second position.

4. An inhaler according to any preceding claim, wherein the inhaler is configured to prevent movement of the retention element from the first position to the second position until the capsule has been ruptured.

5. An inhaler according to any preceding claim, wherein the retention element is configured to substantially prevent movement of the capsule with respect to the capsule receptacle when the retention element is in the first position.

6. An inhaler according to any preceding claim, wherein the capsule receptacle comprises a capsule receptacle opening for allowing the capsule to move between the capsule receptacle and the aerosolization chamber, wherein the retention element is configured to at least partially cover the capsule receptacle opening when the retention element is the first position.

7. An inhaler according to any preceding claim, wherein the retention element comprises a first portion and a second portion, wherein the first portion is positioned within the housing when the retention element is in the first position and the second portion is positioned outside of the housing when the retention element is in the first position.

8. An inhaler according to claim 7, wherein the second portion is angled with respect to the first portion to prevent the second portion from being inserted into the housing when the retention element is in the first position.

9. An inhaler according to any preceding claim, wherein the retention element is configured to substantially prevent airflow through the airflow path when the retention element is in the first position.

10. An inhaler according to any one of claims 1 to 9, wherein the retention element is moved from the first position to the second position by at least partially withdrawing the retention element from the housing through the inlet.

11. An inhaler according to any one of claims 1 to 9, wherein the retention element is moved from the first position to the second position by at least partially withdrawing the retention element from the housing through the outlet.

12. An inhaler according to any preceding claim comprising a porous element positioned in the airflow path between the aerosolization chamber and the outlet, wherein the retention element comprises one or more protruding members that are configured to extend through the porous element when the retention element is in the first position.

13. An inhaler according to any preceding claim comprising a cover, wherein the cover is movable between a cover position in which the outlet is at least partially covered and an uncover position in which the outlet is uncovered, wherein movement of the cover from the cover position to the uncover position is configured to move the retention element from the first position to the second position.

14. An inhaler according to any preceding claim comprising a capsule positioned within the capsule receptacle, the capsule comprising a composition, wherein the composition comprises a pharmaceutically active ingredient and wherein the pharmaceutically active ingredient is a salicylate (a salt or ester of salicylic acid), preferably acetylsalicylic acid or a pharmaceutically acceptable salt thereof.

15. A composition comprising acetylsalicylic acid or a pharmaceutically acceptable salt thereof for use in a method of treating, preventing or ameliorating a thromboembolic event in a patient, wherein the composition is administered to the patient using the inhaler of any one of claims 1 to 14 having a capsule positioned within the capsule receptacle and the capsule comprising the composition, by:
rupturing the capsule with the rupturing element;
moving the retention element from the first position to the second position; and drawing on the outlet to inhale the composition.
